(19) Europäisches Patentamt
European Patent Office
Office européen des brevets

(11) **EP 1 977 791 A1**

(12) **EUROPEAN PATENT APPLICATION**
published in accordance with Art. 158(3) EPC

(43) Date of publication:
08.10.2008 Bulletin 2008/41

(51) Int Cl.:
*A63B 23/18* (2006.01)     *A61B 5/08* (2006.01)
*A63B 24/00* (2006.01)     *A63B 69/00* (2006.01)
*A61M 21/02* (2006.01)

(21) Application number: 06843288.9

(22) Date of filing: 26.12.2006

(86) International application number:
PCT/JP2006/325904

(87) International publication number:
WO 2007/083495 (26.07.2007 Gazette 2007/30)

(84) Designated Contracting States:
**DE ES FR GB IT NL**

(30) Priority: **20.01.2006 JP 2006012882**

(71) Applicants:
• **Omron Healthcare Co., Ltd.**
**Kyoto 615-0084 (JP)**
• **Jichi Medical University**
**Tochigi, 3290498 (JP)**

(72) Inventors:
• **SHIRASAKI, Osamu,**
**Omron Healthcare Co., Ltd.**
**Kyoto-shi,**
**Kyoto 615-0084 (JP)**

• **SHIGA, Toshikazu,**
**Omron Healthcare Co., Ltd.**
**Kyoto-shi,**
**Kyoto 615-0084 (JP)**
• **NAKASE, Yuzo,**
**Omron Healthcare Co., Ltd.**
**Kyoto-shi,**
**Kyoto 615-0084 (JP)**
• **KARIO, Kazuomi,**
**Jicji Medical University**
**Shimotsuke-shi,**
**Tochigi 329-0498 (JP)**

(74) Representative: **Wilhelms · Kilian & Partner**
**Patentanwälte**
**Eduard-Schmid-Strasse 2**
**81541 München (DE)**

(54) **RESPIRATION TRAINING MACHINE ENABLING GRASP OF RESULT**

(57)     A breathing exerciser includes: a guide unit (201) for guiding an exercise pattern of breathing to a user; a breathing sensor for detecting breathing of the user; a breathing index calculating unit (203) for calculating, based on a signal from the breathing sensor, a breathing index at least one of before and after an exercise period during which the exercise pattern is guided, the breathing index representing a characteristic of a breathing state of the user; and an outcome index calculating unit (204) for calculating an outcome index based on at least two breathing indices, the outcome index representing an outcome of breathing exercise; and an informing unit (206) for informing the outcome index to the user.

Fig. 3

EP 1 977 791 A1

**Description**

TECHNICAL FIELD

The present invention relates to a breathing exerciser and more particularly to a breathing exerciser that guides an exercise pattern of breathing.

BACKGROUND ART

It has been verified that slow deep breathing leads to suppression of autonomic nerves, providing an effect of decreasing blood pressure. For example, the document "Slow Breathing Improves Arterial Baroreflex Sensitivity and Decreases Blood Pressure in Essential Hypertension" (hereinafter, referred to as the "Non-Patent Document 1 ") by Chacko N.Joseph, et al., in "Hypertension, October 2005", Volume 46, pp. 714-718, published by the American Heart Association, can be a reference document. Therefore, conventionally, as autonomic nervous system exercise methods and biofeedback, studies have been conducted. Also, a number of breathing exercisers for that have been proposed.
Japanese Patent Application Laid-Open No. 62-277976 (hereinafter, referred to as the "Patent Document 1") has disclosed an invention related to an abdominal breathing exercising apparatus in which a sensor that detects movement of the abdominal cavity caused by subject's abdominal breathing is placed on his/her abdomen, a predetermined ideal breathing exercise pattern is generated, an actual berating pattern is compared with the ideal breathing exercise pattern to determine the degree of matching, and a result of the determination is informed by sound or photoelectric display. Also, Published Japanese Translation of PCT Application No. 2005-535378 (hereinafter, referred to as the "Patent Document 2") has disclosed an invention of a breathing exerciser including a signal generating apparatus that determines a breathing pattern by an input from a breathing sensor.
It has become clear that respiratory standstill during sleep due to airway obstruction or autonomic nervous system abnormalities, which is a so-called "Sleep Apnea Syndrome (SAS)", not only simply reduces sleep quality, causing drowsiness during daytime active hours but also promotes hypertension and thereby induces harmful blood pressure fluctuations, which becomes a cause of many serious diseases such as heart diseases and brain diseases.
As treatment approaches for the SAS, there have been proposed an apparatus (CPAP) that delivers a positive pressure of air to the obstructed airway, a surgical operation for expanding the airway, medical treatment (application of an alveolar surfactant preparation to the posterior region of the pharynx (see Published Japanese Translation of PCT Application No. 2001-507364 (hereinafter, referred to as the "Patent Document 3")), enhancement of the muscle groups by muscle strength stimulation exercise by low frequency vibration of the muscle groups of the tongue root in the cervical region (see Japanese Patent Application Laid-Open No. 2005-237807 (hereinafter, referred to as the "Patent Document 4")), etc. However, any of the approaches is a great burden for patients and the current state is that low-burden approaches to the prevention of the SAS have not been proposed.
Therefore, for the prevention of the SAS also, breathing exercise that patients can easily do is considered to be useful.

[Patent Document 1] Japanese Patent Application Laid-Open No. 62-277976
[Patent Document 2] Published Japanese Translation of PCT Application No. 2005-535378
[Patent Document 3] Published Japanese Translation of PCTApplication No. 2001-507364
[Patent Document 4] Japanese Patent Application Laid-Open No. 2005-237807
[Non-Patent Document 1] Chacko N.Joseph, Cesare Porta, Gaia Casucci, Nadia Casiraghi, Mara Maffeis, Marco Rossi, Luciano Bernardi, "Slow Breathing Improves Arterial Baroreflex Sensitivity and Decreases Blood Pressure in Essential Hypertension", "Hypertension, October 2005", the American Heart Association, Volume 46, pp. 714-718

DISCLOSURE OF THE INVENTION

PROBLEMS TO BE SOLVED BY THE INVENTION

However, both of the Patent Documents 1 and 2 have disclosed techniques for merely approximating an actual breathing state to an ideal breathing pattern and information from a breathing sensor is not used as an information source for checking an outcome brought about by exercise. Therefore, there is a problem that the user cannot (instantly) grasp an in-situ outcome brought about by exercise, leading to a decrease in motivation to continue breathing exercise.
The present invention is made to solve problems such as those described above and an object of the present invention is therefore to provide a breathing exerciser that enables a user to easily grasp an effect brought about by breathing exercise.

MEANS FOR SOLVING THE PROBLEMS

A breathing exerciser according to one aspect of the present invention comprises: a guide unit for guiding an exercise pattern of breathing to a user; a detecting unit for detecting breathing of the user; a first calculating unit for calculating, based on a signal from the detecting unit, a breathing index at least one of before and after an exercise period during which the exercise pattern is guided, the breathing index representing a characteristic of a breathing state of the user; a second calculating unit for calculating an outcome index based on at least two breathing indices, the outcome index representing an outcome of breathing exercise; and an informing unit for informing the outcome index to the user.

Preferably, the first calculating unit calculates two breathing indices based on breathings respectively detected before and after the exercise period, and the second calculating unit calculates an outcome index for before and after the exercise period, based on the calculated breathing indices.

Preferably, the breathing exerciser further comprises: a storage unit for storing information on a breathing index which is based on breathing detected before a previous exercise period, the first calculating unit calculates a breathing index based on breathing detected before a current exercise period, and the second calculating unit calculates an outcome index for before each exercise period, based on the breathing index calculated by the first calculating unit and the information on a breathing index stored in the storage unit.

Preferably, the breathing exerciser further comprises: a storage unit for storing information on a breathing index which is based on breathing detected after a previous exercise period, the first calculating unit calculates a breathing index based on breathing detected after a current exercise period, and the second calculating unit calculates an outcome index for after each exercise period, based on the breathing index calculated by the first calculating unit and the information on a breathing index stored in the storage unit.

Preferably, the first calculating unit further calculates a plurality of breathing indices based on breathings detected for a plurality of times during the exercise period, and the second calculating unit further calculates an outcome index for during the exercise period, based on the breathing indices for during the exercise period.

Preferably, the breathing exerciser further comprises: a changing unit for changing the exercise pattern based on a result of comparison between the outcome index for during the exercise period which is calculated by the second calculating unit and a predetermined threshold.

A breathing exerciser according to another aspect of the present invention comprises: a guide unit for guiding an exercise pattern of breathing to a user; a detecting unit for detecting breathing of the user; a first calculating unit for calculating, based on a signal from the detecting unit, a breathing index at least one of before and after an exercise period during which the exercise pattern is guided, the breathing index representing a characteristic of a breathing state of the user; and an informing unit for informing at least two breathing indices to the user.

Preferably, the breathing index is one of a breathing cycle or a number of breaths, a balance between an exhalation operation and an inhalation operation, breathing flow rate, uniformity of the breathing flow rate, and silent property.

More preferably, the breathing exerciser further comprises: a storage unit for storing an outcome index associated with a previous exercise period, and the informing unit further informs a trend between the stored outcome index and the calculated outcome index.

More preferably, the first calculating unit calculates a plurality of breathing indices and the breathing exerciser further comprises a third calculating unit for calculating an ideal breathing index based on the plurality of calculated breathing indices and a predetermined computational expression.

EFFECT OF THE INVENTION

According to the present invention, the user can easily grasp an in-situ outcome brought about by breathing exercise. By this, it becomes possible to improve motivation to continue breathing exercise.

BRIEF DESCRIPTION OF THE DRAWINGS

FIG. 1 is a diagram showing a schematic view of a breathing exerciser in an embodiment of the present invention.
FIG. 2 is a block diagram showing a configuration of an exerciser main body of the breathing exerciser in the embodiment of the present invention.
FIG. 3 is a functional block diagram showing functions of a CPU in the breathing exerciser in the embodiment of the present invention.
FIG. 4 is a diagram showing an exemplary structure of an exercise result storage unit in the embodiment of the present invention.

FIG. 5 is a flowchart showing a flow of a breathing exercise process in the embodiment of the present invention.

FIG. 6 is a diagram showing an example of a screen to be displayed when inputting physical information.

FIG. 7 is a diagram showing an exemplary display of a breathing guide.

FIG. 8 is a diagram for describing a detailed exemplary display of the breathing guide based on an exercise pattern for during an exercise period.

FIG. 9 is a diagram showing an exemplary display of information on an outcome index for before and after exercise.

FIG. 10 is a diagram showing an exemplary display of a trend of outcome indices.

FIG. 11 is a diagram showing an exemplary screen display showing a relationship between values of two breathing indices and a target value, for the case in which the breathing index is of one type.

FIG. 12 is a diagram showing an exemplary screen display showing a relationship between values of two breathing indices and a target value, for each type of breathing index.

DESCRIPTION OF REFERENCE NUMERALS

1: exerciser main body, 2: breathing sensor, 3: wiring line, 4: display unit, 5: driver, 10: I/O, 12: memory, 13: timer, 15: A/D converter, 20: CPU, 21: operation unit, 24: speaker, 25: amplifier, 100: breathing exerciser, 122: pattern storage unit, 126: exercise result storage unit, 201: guide unit, 202: breathing information obtaining unit, 203: breathing index calculating unit, 204: outcome index calculating unit, and 206: informing unit.

BEST MODE FOR CARRYING OUT THE INVENTION

Embodiments of the present invention will be described in detail with reference to the drawings. Note that the same or corresponding parts are denoted by the same reference numerals throughout the drawings.

[First Embodiment]

(For configuration)

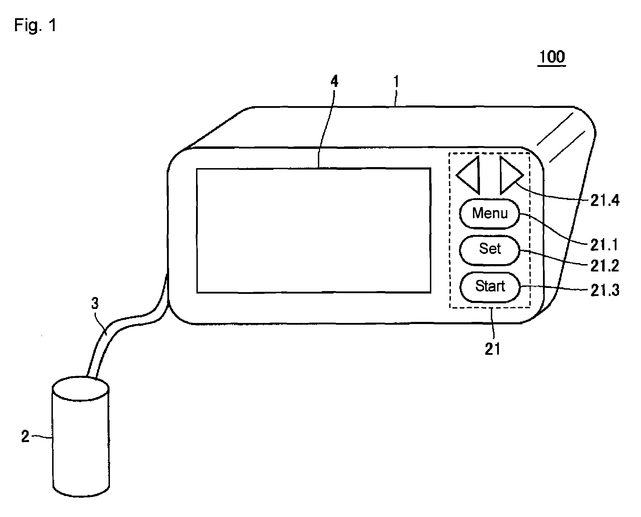

FIG. 1 is a diagram showing a schematic view of a breathing exerciser 100 in a first embodiment of the present invention.

Referring to FIG. 1, the breathing exerciser 100 according to the present embodiment includes an exerciser main body 1; a breathing sensor 2 for detecting user's breathing; and a wiring line 3 for supplying a signal from the breathing sensor 2 into the exerciser main body 1.

The breathing sensor 2 is a mouthpiece-type sensor having a cylindrical form and detects, for example, the number of turns of a propeller provided inside a cylinder. By this, by the user putting the breathing sensor 2 in his/her mouth and breathing, the flow rate of air (vital capacity) passing through the cylinder can be detected.

The exerciser main body 1 includes a display unit 4 provided so that the user can check display content; and an operation unit 21 provided so that the user can externally operate the exerciser. The display unit 4 is configured by, for example, an LCD (Liquid Crystal Display). The operation unit 21 includes a plurality of switches for accepting an input of an instruction from the user, e.g., a menu switch 21.1 for accepting an instruction to display a menu showing various functions of the breathing exerciser 100; a set switch 21.2 for accepting an instruction to execute each function or each operation; a start switch 21.3 for accepting an instruction to start exercise; left and right scroll switches 21.4; and the like.

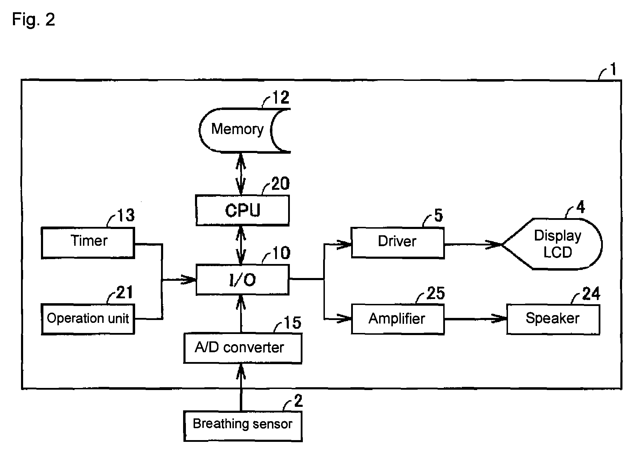

FIG. 2 is a block diagram showing a configuration of the exerciser main body 1 of the breathing exerciser 100 in the embodiment of the present invention. Referring to FIG. 2, the exerciser main body 1 includes a CPU (Central Processing Unit) 20 for intensively controlling and monitoring each unit; a memory 12 in which various data and programs are to be stored; a display unit 4; a driver 5 that controls the display operation of the display unit 4; an operation unit 21; a timer 13 that performs a timer operation to output timer data; an amplifier 25; a speaker 24 for outputting sound through the amplifier 25; an A/D converter 15 for converting an output signal from the breathing sensor 2 from an analog signal to a digital signal; and an input/output interface (I/O) 10 that controls input and output of data within the breathing exerciser 100. Timer data outputted from the timer 13 and information inputted to the operation unit 21 are inputted to the CPU 20 through the I/O 10. The CPU 20 controls the operations of the display unit 4 and the speaker 24 through the I/O 10.

Note that the breathing sensor 2 is not limited to such a configuration as that described above. For example, the breathing sensor 2 may be a micro pressure sensor that can be attached below the user's nose. The micro pressure sensor is a sensor that can also capture a change in air pressure which is changed by movement of air. By attaching the micro pressure sensor below the nose, a measurement can be performed where the influence of disturbance such as the flow of other atmosphere is minimized. Therefore, by attaching the micro pressure sensor so that the flow of air from the nasal cavity can be captured, whether it is an inhalation state or an exhalation state can be distinguished. Alternatively, the breathing sensor 2 may be a temperature sensor. By attaching the temperature sensor below the nose, a breathing state can be detected from a difference in air temperature between exhalation and outside air (inhalation).

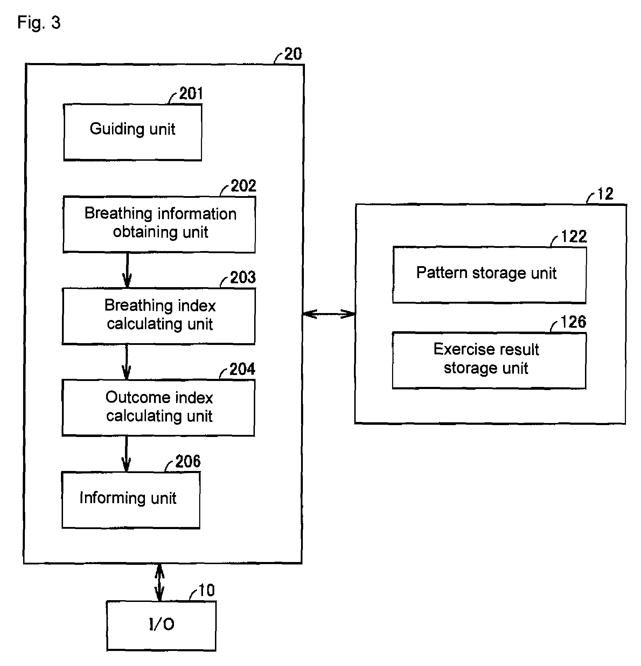

FIG. 3 is a functional block diagram showing functions of the CPU 20 in the breathing exerciser 100 in the embodiment of the present invention.

Referring to FIG. 3, the CPU 20 includes a guide unit 201 for guiding an exercise pattern of breathing to the user; a breathing information obtaining unit 202 for obtaining a detection signal (hereinafter, referred to as "breathing information") from the breathing sensor 2; a breathing index calculating unit 203 for calculating a breathing index representing a characteristic of a user's breathing state, based on the obtained breathing information; an outcome index calculating unit 204 for calculating an outcome index representing an outcome of breathing exercise, based on at least two breathing indices calculated by the breathing index calculating unit 203; and an informing unit 206 for informing the outcome index calculated by the outcome index calculating unit 204 to the user.

The "exercise period" is a period during which an exercise pattern is guided by the guide unit 201. The guide unit 201 specifically performs a process of displaying information (hereinafter, also referred to as a "breathing guide") for guiding to breathing which is based on an exercise pattern, on the display unit 4. Note that although in the present embodiment description is made such that an exercise pattern is guided using the display unit 4, an exercise pattern may be guided by sound by the speaker 24.

The breathing information obtaining unit 202 obtains, as breathing information, information on flow rate from the breathing sensor 2 for a predetermined period of time (e.g., one minute) at predetermined timing. In the present embodiment, breathing information is obtained before the exercise period and after the exercise period.

The breathing index calculating unit 203 calculates, as a breathing index, for example, the number of breaths, i.e., the number of breaths per certain period of time (e.g., one minute), based on the obtained breathing information (information on flow rate). That is, the number of breaths is calculated by applying a predetermined algorithm to the obtained breathing information. For a procedure of calculation of the number of breaths, any known procedure which is conventionally provided can be used and thus a detailed description thereof is omitted here. Note that instead of/in addition to the number of breaths, other breathing indices, e.g., a breathing cycle, a balance between an exhalation operation and an inhalation operation, breathing flow rate, uniformity of breathing flow rate, silent property, etc., may be calculated. These breathing indices can also be calculated using publicly known techniques.

Based on the calculated breathing indices, the outcome index calculating unit 204 calculates outcome indices respectively for before each exercise and for before and after exercise. Each outcome index is specifically calculated as, for example, a difference between two breathing indices or a ratio between two breathing indices. In the present embodiment, it is assumed that as an outcome index the degree of reduction in the number of breaths is calculated.

An outcome index for before exercise is calculated using a breathing index which is based on breathing information detected before (immediately before) a previous exercise period and a breathing index which is based on breathing information detected before (immediately before) a current exercise period. The "previous exercise period" refers to an exercise period in a breathing exercise process which is performed earlier than a current breathing exercise process, and may be, for example, the first exercise period or may be the last exercise period. In the following description, it is assumed that an outcome index for before each exercise is calculated based on a breathing index obtained before the first exercise period and a breathing index obtained before a current exercise period.

An outcome index for before and after exercise is calculated using a breathing index which is based on breathing information detected before (immediately before) the current exercise period and a breathing index which is based on breathing information detected after the current exercise period (after a predetermined period of time).

Although in the present embodiment outcome indices are thus calculated before each exercise and before and after exercise, at least one of such outcome indices may be calculated. Alternatively, in addition to them/instead of them, for example, an outcome index for after each exercise may be calculated. Namely, an outcome index for after each exercise may be calculated using a breathing index which is based on breathing information detected after a previous exercise period (after a predetermined period of time) and a breathing index which is based on breathing information detected after the current exercise period (after a predetermined period of time). Alternatively, an outcome index for during the exercise period may be calculated based on a plurality of pieces of breathing information detected during the exercise period, or an outcome index may be calculated based on a breathing index obtained before the current exercise period and a breathing index obtained during the current exercise period.

The CPU 20 may further calculate a change (trend) of the outcome of breathing exercise, based on a difference or ratio between a past outcome index and a current outcome index. Alternatively, a current target value may be computed based on a predetermined computational expression and a past outcome index to calculate a shift of a current outcome index from the target value.

The informing unit 206 specifically performs a process of displaying information on the calculated outcome indices, on the display unit 4. The informing unit 206 may further display a trend of the outcome indices on the display unit 4.

Note that the operations of the functional blocks in the CPU 20 may be implemented by executing software stored in the memory 12 or at least one of them may be implemented by hardware.

The memory 12 is a non-volatile memory, e.g., flash memory. The memory 12 includes a pattern storage unit 122 in which data on a plurality of exercise patterns is stored in advance; and exercise result storage unit 126 for storing exercise results. Note that the storage units do not need to be included in the same storage medium (memory 12) and may be included in different storage media.

In the pattern storage unit 122, a plurality of exercise pattern data with different load levels are stored in advance.

The exercise pattern data includes information on at least exercise time (exercise period) or the number of breaths, a breathing cycle, and the depth of breathing. The load level is determined by, for example, at least one of parameters including at least exercise time or the number of breaths, a breathing cycle, and the depth of breathing. In the present embodiment, it is assumed that the load level is determined by a breathing cycle (the number of breaths per certain period of time).

Each exercise pattern data may include a plurality of pattern data with different load levels. Namely, a plurality of patterns with different parameters of at least one type (e.g., the depth of breathing) may be included in each exercise pattern.

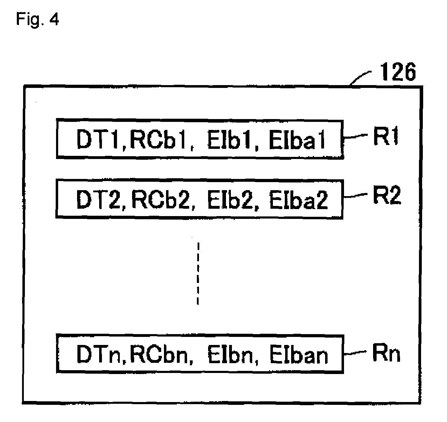

Next, a specific example of a data structure in the exercise result storage unit 126 will be described with reference to FIG. 4. Referring to FIG. 4, in the exercise result storage unit 126, exercise results are stored in the unit of record R. The record R includes date and time data DT indicating exercise data and time; number-of-breaths data RC indicating the number of breaths (breathing index) for before exercise; outcome index data Elb indicating the degree of reduction in the number of breaths (outcome index) for before each exercise; and outcome index data Elba indicating the degree of reduction in the number of breaths (outcome index) for before and after exercise. Note that a storage format is not limited to the one using the record R and can be any as long as such data is stored so as to be associated with each exercise.

(For operation)

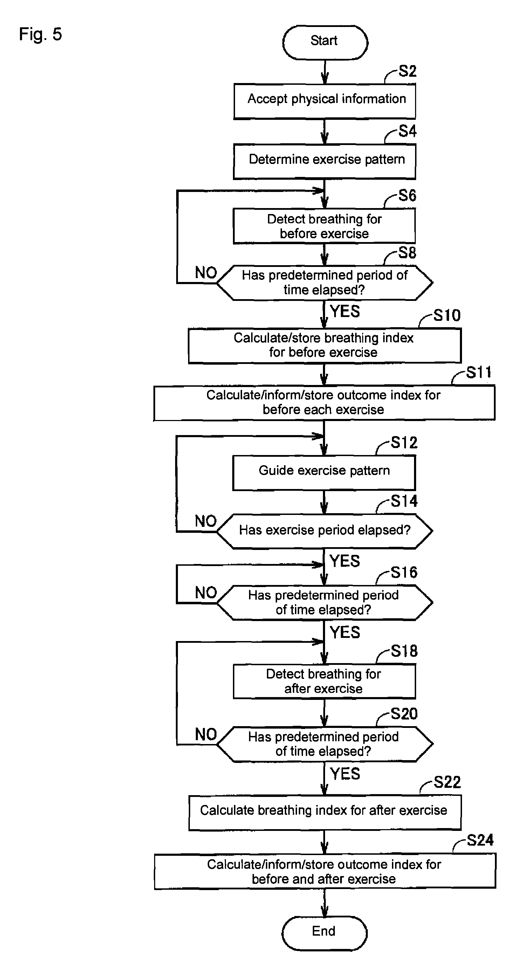

A breathing exercise process in the embodiment of the present invention shown in a flowchart in FIG. 5 is stored in advance in the memory 12 as a program and a function of the breathing exercise process is implemented by the CPU 20 reading and executing this program. Note that for simplification of description it is assumed that a series of current breathing exercise process is an nth one (n: natural number).

Referring to FIG. 5, first, the CPU 20 accepts an input of physical information from the user (step S2). The physical information is information indicating a user's physical characteristic and includes at least one of, for example, a blood pressure value, height, weight, age, and sex. Here, it is assumed that information on height and weight is accepted as physical information.

Then, based on the accepted physical information, an exercise pattern to be guided to the user in this breathing exercise process is determined (step S4). Specifically, for example, a process such as that shown below is performed. An association table in which height and weight are associated with identification information on an exercise pattern is stored in advance in, for example, the pattern storage unit 122. The CPU 20 identifies identification information associated with the user's height and weight in the association table. In this manner, an exercise pattern indicated by the identified identification information is determined as a guide target exercise pattern. The CPU 20 reads data on the guide target exercise pattern from the pattern storage unit 122.

Then, user's breathing is detected by the breathing sensor 2 (step S6). That is, the breathing information obtaining unit 202 obtains breathing information from the breathing sensor 2. The obtaining of breathing information is performed for a predetermined period of time (e.g., one minute) (NO in step S8). If it is determined that the predetermined period of time has elapsed from the start of detection of breathing (YES in step S8), then processing proceeds to step S10.

In step S10, the breathing index calculating unit 203 performs a calculation/storage process of a breathing index for before exercise, based on the breathing information detected before exercise. Namely, the breathing index calculating unit 203 calculates the number of breaths based on the breathing information detected in step S6. Then, data on the calculated number of breaths (data on the breathing index) is stored in the exercise result storage unit 126 as number-of-breaths data RCn. Note that at the time of detecting breathing or before the detection, the user may be urged to do normal breathing (not to take a deep breath or a quick breath).

Subsequently, a calculation/informing/storage process of an outcome index for before each exercise is performed (step S11). Specifically, the outcome index calculating unit 204 calculates an outcome index, i.e., the degree of reduction in the number of breathes, for before each exercise, based on the number-of-breaths data RCn for the current time which is calculated and stored in step S10 and number-of-breaths data RC1 for the first time. Information indicating the calculated degree of reduction in the number of breaths is displayed in a predetermined area of the display unit 4. By this, the degree of reduction in the number of breaths for before the current exercise with reference to the number of breaths for before the first exercise can be informed to the user. Furthermore, the calculated degree of reduction in the number of breaths for before each exercise is stored as outcome index data Elbn.

Next, the guide unit 201 guides the exercise pattern determined in step S4 to the user (step S12). Specifically, based on the data on the exercise pattern read in step S4, a breathing guide (e.g., how much more exhalation and inhalation should be performed) is displayed on the display unit 4. The process in step S12 is performed until the exercise period has elapsed (NO in step S14).

Note that although in the present embodiment after the process in step S11 processing automatically transitions to the exercise period, processing may be transition to the exercise period when an instruction to start exercise from the user is inputted.

Also, during the exercise period too, detection of breathing and calculation of a breathing index may be performed at predetermined timing. Alternatively, an outcome index may be further calculated based on two calculated breathing indices for during the exercise period. In such a case, the exercise pattern being executed may be changed based on the breathing indices or outcome index for during the exercise period. Specifically, for example, when, as described above, each exercise pattern includes a plurality of patterns and a setting is such that the load level of the pattern increases along the time axis, if the breathing index or outcome index exceeds a predetermined upper limit, then a pattern with a next load level may be skipped. That is, when the breathing index or outcome index exceeds the upper limit, a pattern with a two-step higher load level than that of the pattern being executed may be executed. When the breathing index or outcome index falls below a predetermined lower limit, the pattern being executed may be repeated. Note that these processes can be performed by publicly known techniques, e.g., using a pointer. By thus dynamically changing the exercise pattern based on the value of the breathing index or outcome index for during the exercise period, a breathing guide according to the degree of achievement of the user can be displayed. Note that when detection of breathing is thus performed also during the exercise period, it is desirable that the breathing sensor 2 is of a type that is attached below the user's nose (e.g., a micro pressure sensor), rather than of a mouthpiece-type.

If in step S14 it is determined that the exercise period has elapsed (YES in step S14), then processing proceeds to step S16. In step S16, it is determined whether a predetermined period of time (e.g., two minutes) has elapsed after the exercise period is finished, and processing waits until the predetermined period of time has elapsed (NO in step S16). It is desirable that the predetermined period of time here is such a period of time that is assumed to be required for the user to return to a normal breathing state after ending the breathing exercise.

If the predetermined period of time has elapsed (YES in step S16), then user's breathing is detected by the breathing sensor 2 (step S18). As is the case with before exercise, here also, the breathing information obtaining unit 202 obtains breathing information until a predetermined period of time (e.g., one minute) has elapsed from the start of the detection of breathing (NO in step S20). If it is determined that the predetermined period of time has elapsed from the start of the detection of breathing (YES in step S20), then processing proceeds to step S22.

In step S22, the breathing index calculating unit 203 performs a calculation process of a breathing index for after exercise, based on the detected breathing information. That is, the breathing index calculating unit 203 calculates the number of breaths based on the breathing information detected in step S18. Note that at the time of detecting breathing or before the detection, the user may be urged to do normal breathing.

Subsequently, a calculation/informing/storage process of an outcome index for before and after exercise is performed (step S24). Specifically, the outcome index calculating unit 204 calculates an outcome index, i.e., the degree of reduction in the number of breaths, for before and after exercise, based on the breathing index (the number of breaths) for before exercise which is calculated in step S10 and the breathing index (the number of breaths) for after exercise which is calculated in step S22. Information on the calculated degree of reduction in the number of breaths is displayed in a predetermined area of the display unit 4. By this, the degree of reduction in the number of breaths for after exercise with reference to the number of breaths for before exercise can be informed to the user. Furthermore, the calculated degree of reduction in the number of breaths for before and after exercise is stored as outcome index data Elban.

With the above, the breathing exercise process ends.

Note that it is desirable that the user is able to view, by a predetermined operation, information on outcome indices stored in the exercise result storage unit 126 in the above-described breathing exercise process. Specifically, for example, by performing an operation such as that shown below, information on past outcome indices can be provided to the user. Namely, for example, by the user pressing the menu switch 21.1, information on a plurality of functions including "viewing of outcomes" is displayed on the display unit 4. Then, by the user operating the scroll switches 21.4 and the set switch 21.2 to select the function of viewing of outcomes, by the CPU 20 the latest outcome index data Elbn and Elban stored in the exercise result storage unit 126 are read and displayed on the display unit 4. Furthermore, by operating the scroll switches 21.4, past outcome index data Elbn and Elban may be sequentially read and displayed on the display unit 4.

Alternatively, instead of this/in addition to this, after the process in step S24, a trend of exercise indices may be automatically displayed. That is, when there are past outcome indices (outcome indices associated with a previous exercise period), the informing unit 206 displays a trend between the past outcome indices and current outcome indices on the display unit 4.

(For exemplary display)

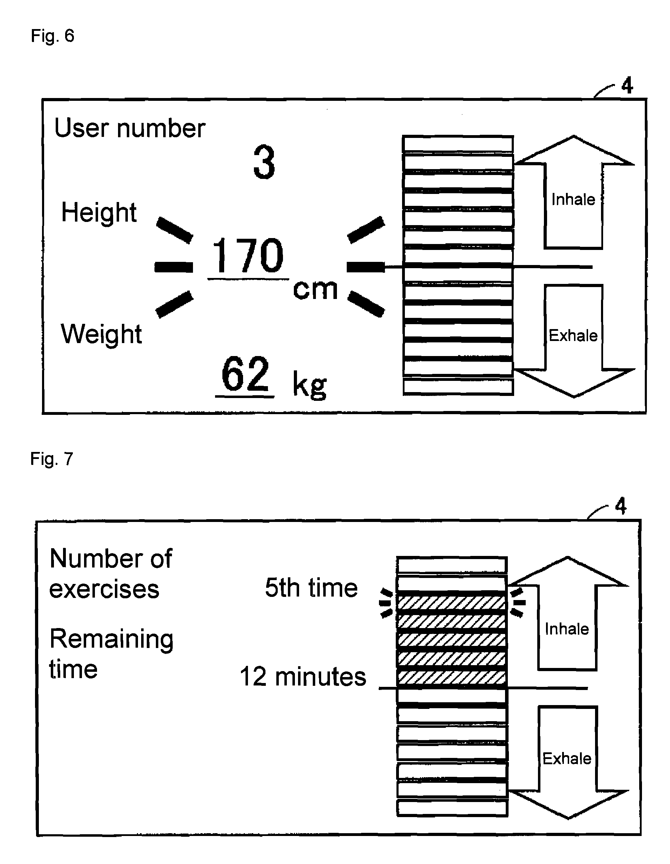

FIG. 6 is a diagram showing an example of a screen to be displayed when inputting physical information in step S2. As shown in FIG. 6, an item (height or weight) of physical information being inputted is displayed blinking. The physical information can be inputted using the scroll switches 21.4 and the set switch 21.2. Note that in order that a plurality of users can use the exerciser a user number may be inputted. In this case, exercise results are stored so as to be associated with the inputted user number.

FIG. 7 is a diagram showing an exemplary display of a breathing guide in step S12. Referring to FIG. 7, a breathing guide is performed by highlighting/not highlighting 14 blocks displayed in a vertical direction on the screen. Also, the number of exercises (nth time) and remaining time are displayed in their respective predetermined areas.

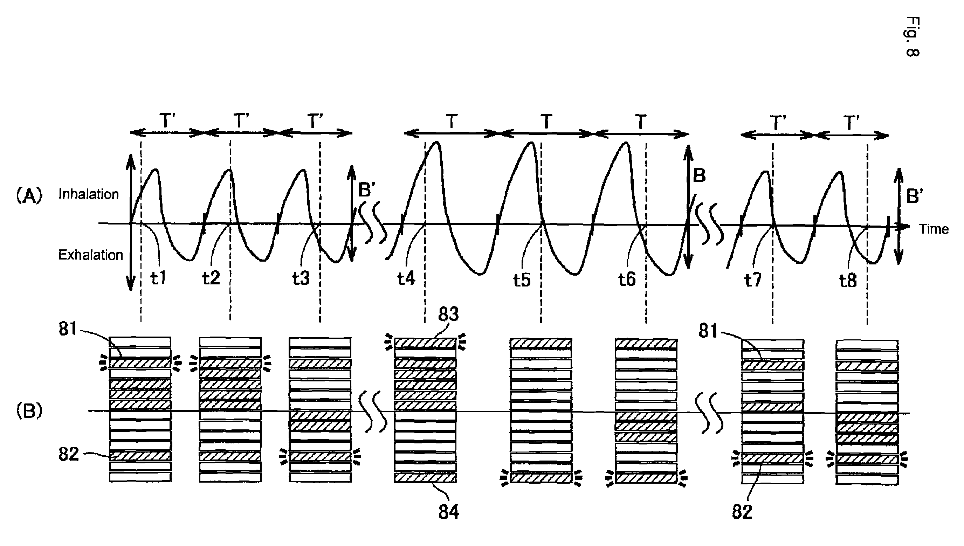

Detailed exemplary display of the breathing guide will be described with reference to FIG. 8. (A) of FIG. 8 is a diagram showing an example of an exercise pattern and (B) is a diagram showing an exemplary display of the breathing guide at arbitrary times t1 to t8 of the exercise pattern shown in (A).

Referring to (A) of FIG. 8, an exercise period based on the exercise pattern shown here includes a warm-up period, a substantial exercise period, and a cool-down period. That is, the exercise pattern includes a plurality of patterns.

Referring to (B) of FIG. 8, in each breathing guide, a block at a location indicating the depth of breathing is fixedly displayed highlighted (displayed darkened). In the guides at times t1, t2, and t3 during the warm-up period, fifth blocks 81 and 82 respectively located upward and downward from the center are fixedly displayed highlighted. In the breathing guide at time t1, the first to third blocks located upward from the center are displayed highlighted and the block 81 is displayed blinking. By this, it is possible to inform how much more time he/she should inhale to the user. In the breathing guide at time t2, similarly, the block 81 is displayed blinking and all of the first to fourth blocks located upward from the center are displayed highlighted. By this, it is possible to inform that it is the end of an inhalation period.

At times t4, t5, and t6 during the substantial exercise period, seventh blocks (blocks at both ends) 83 and 84 respectively located upward and downward from the center are fixedly displayed highlighted. In the breathing guide at time t5, the block 84 is displayed blinking and blocks other than the block 83 are displayed not highlighted (displayed blanked). By this, it is guided that inhalation is finished and thus the user should move to exhalation.

At times t7 and t8 during the cool-down period, as with the warm-up period, fifth blocks 81 and 82 respectively located upward and downward from the center are fixedly displayed highlighted. In the breathing guide at time t7, the block 82 is displayed blinking and the first block located upward from the center is displayed highlighted. By this, it is guided that an exhalation state should be continued for on the order of another one-half.

Note that the display mode of the breathing guide is not limited to highlight/no highlight such as that described above; for example, the display color of blocks may be changed.

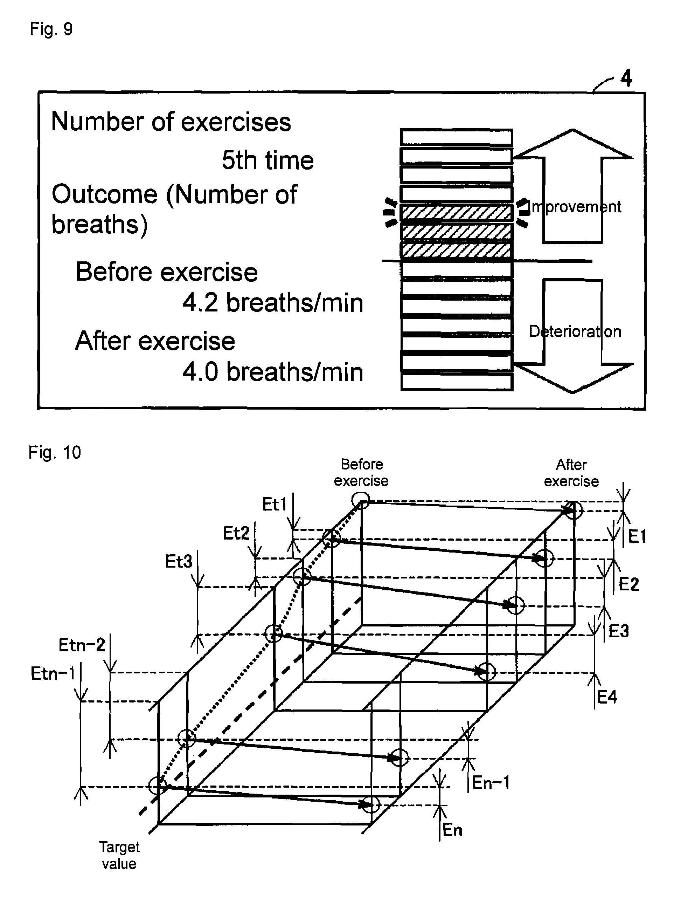

Referring to FIG. 9, the outcome index is, for example, level-displayed. Level-display is performed by highlighting/not highlighting 14 blocks, as with the breathing guide. Of the 14 blocks, seven blocks displayed on the upper side represent improvement and seven blocks displayed on the lower side represent deterioration. Note that it is assumed that levels (level -7 to level +7) are predetermined according to the value of the degree of reduction in the number of breaths.

In FIG. 9, among the seven blocks located on the upper side, the first and second blocks from the center are displayed highlighted (displayed darkened) and the third block from the center is displayed blinking. By this, the user is informed that the number of breaths for after exercise is reduced (improved) by three levels, as compared with the number of breaths for before exercise. In this case, it is desirable to further display the number of breaths (breathing index) for before exercise and the number of breaths (breathing index) for after exercise. By this, the user can grasp the degree of improvement/deterioration in breathing index (the number of breaths) in greater detail.

Note that although here information on the outcome index is informed by the level of improvement/deterioration, the informing form is not limited thereto; for example, the value of the outcome index (the degree of reduction in the number of breaths) itself may be informed. When displaying information on the outcome index, the number of exercises may be further displayed.

In FIG. 10, there are displayed a trend of outcome indices for before each exercise and a trend of outcome indices for before and after exercise, for the first time to the current time. In the drawing, E1, E2,..., En each represent an outcome index for before and after exercise, i.e., an outcome per exercise. Et1, Et2,..., Etn each represent an outcome index for before each exercise, i.e., a cumulative outcome of exercise according to the progression of exercise. By the trends of outcome indices thus being displayed, the user can visually grasp short-term/long-term outcomes of exercise.

Note that instead of showing trends of outcome indices for the first time to the current time, trends of outcome indices for a certain period of time (e.g., for one week) may be displayed.

An outcome index for during exercise or for after each exercise may be further displayed. Alternatively, the user may be allowed to select an outcome index which he/she wants to be displayed and a trend of the selected outcome index may be displayed.

As described above, in the present embodiment, an outcome index representing an outcome of breathing exercise is informed to the user. However, as long as the user can grasp an outcome of breathing exercise, for example, instead of an outcome index, two breathing indices (e.g., a breathing index for before the first exercise and a breathing index for before current exercise, etc.) may be informed to the user. In this case, the outcome index calculating unit 204 in FIG. 3 may not be included in the CPU 20. Also, instead of step S11 in FIG. 5, a breathing index for before current exercise calculated in step S10 and a breathing index for before the first exercise are informed. Specifically, for example, these two breathing indices are simultaneously or alternately displayed on the display unit 4. Similarly, instead of step S24, a breathing index for before current exercise calculated in step S10 and a breathing index for after current exercise calculated in step S22 may be informed.

When the informing unit 206 informs two breathing indices, a target value of the breathing index (ideal breathing index) may be further informed along with the two breathing indices. Such a target value is calculated (determined) based on, for example, user's physical information and a predetermined computational expression or a table. Exemplary screen display for this case is shown in FIG. 10. It is assumed that such a target value for each physical information is predetermined by, for example, clinical experiment, etc.

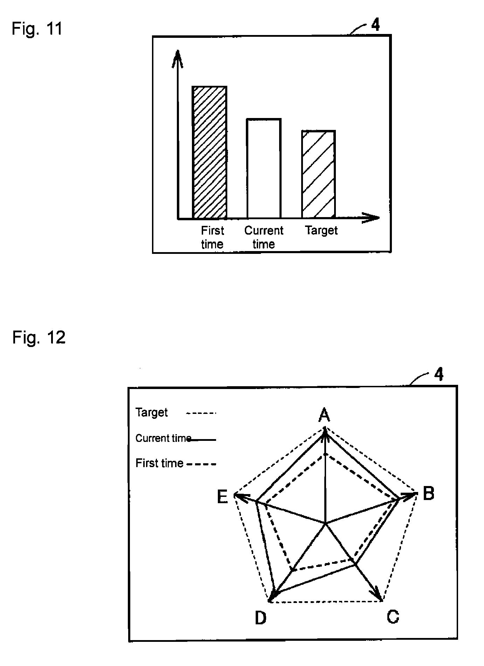

FIG. 11 is a diagram showing an exemplary screen display showing a relationship between values of two breathing indices and a target value.

Referring to FIG. 11, in the case of one type of breathing index, bar graphs respectively corresponding to a value of a breathing index for the first time, a value of a breathing index for the current time, and the target value are displayed side by side. By this, the user can easily grasp how much closer he/she is to the target value. Since each value is shown by graph-display, an outcome of breathing exercise can be visually grasped. Note that although here pieces of information on the respective values are simultaneously shown, the pieces of information on the respective values may be selectively switched and displayed.

Note also that although, as described above, in the present embodiment description is made such that one type of breathing index and an outcome index for the one type of breathing index are calculated, two or more types of breathing indices and outcome indices respectively for the breathing indices may be calculated and two or more types of outcome indices may be informed to the user. That is, the breathing index calculating unit 203 may (simultaneously) calculate two or more types of breathing indices (e.g., the number of breaths and breathing flow rate, etc.) based on detected breathing information. Alternatively, the outcome index calculating unit 204 may calculate outcome indices based on two values calculated for each type (of breathing index).

Alternatively, when two or more types of breathing indices are calculated, information on two values (e.g., the number of breaths for before exercise and the number of breaths for after exercise; breathing flow rate for before exercise and breathing flow rate for after exercise; etc.) may be informed for each type. In such a case too, a relationship between values of breathing indices and a target value may be further shown for each type. Exemplary display for this is shown in FIG. 12.

As shown in FIG. 12, when there are a plurality of types of breathing indices, a relationship between values of two breathing indices and a target value for each type may be displayed in graph form so that the degree of achievement of outcomes can be seen. Here, an example is shown of the case of using breathing indices A to E. It is assumed that the breathing indices are, for example, as follows.

Breathing index A: The number of breaths or a breathing cycle,

Breathing index B: Time balance between an exhalation operation and an inhalation operation,

Breathing index C: Breathing flow rate (vital capacity),

Breathing index D: Uniformity of breathing flow rate (stable breathing), and

Breathing index E: Silent property of breathing (silent breathing).

The breathing index A represents the time of one cycle of an exhalation operation and an inhalation operation and it is desirable that the value is larger. The breathing index B represents the ratio between exhalation time and inhalation time and it is desirable that both have the same time. The breathing index B may be calculated by, for example, a difference between the exhalation time and the inhalation time. The breathing index C represents vital capacity which is measured from an amount upon exhalation (the amount the user breathes out) and it is desirable that the value is larger. The breathing index D represents the uniformity of air flow rate during inhalation time and it is desirable that stable time is longer. The breathing index D represents, for example, time during which flow rate is within a range of 10% higher or lower than average flow rate in one breathing cycle and it is desirable that the value is larger. The breathing index E represents noise that occurs from mouth or nose upon breathing and it is desirable that the value is smaller. Note that to calculate all these breathing indices it is preferable that the breathing sensor 2 includes a mouthpiece-type sensor and a microphone.

Also, based on at least two of the above-described breathing indices A to E, the CPU 20 may further calculate an ideal breathing index representing how much closer the user's breathing state is to an ideal breathing state. The ideal breathing index is calculated by, for example, a computational expression such as that shown below.

$$\text{Ideal breathing index} = (1-A')+B'+(1-C')+(1-D')+E'/30$$

A'= One cycle time (breathing index A)/Standard time
B'= Difference between exhalation time and inhalation time (breathing index B)/One cycle time (breathing index A)
C'= Vital capacity (breathing index C)/Standard vital capacity
D'= Time during which flow rate is within a range of 10% higher or lower than average flow rate in one breathing cycle (breathing index D)/One cycle time

(breathing index A)

$$E' = \text{Noise volume dB (breathing index E)-Standard noise volume dB}$$

It can be considered that the smaller the value of the ideal breathing index computed by the above-described computational expression, the closer it is to the ideal breathing state. Note that it is preferable that the above-described computational expression for the ideal breathing index is predetermined based on clinical experiment, etc. In the computational expression for the ideal breathing index, the coefficient may be increased according to the weight of each value (breathing index). Two calculated ideal breathing indices (e.g., an ideal breathing index for before exercise and an ideal breathing index for after exercise) may be informed to the user by the informing unit 206.

Note that in the present embodiment description is made such that data on a plurality of exercise patterns is stored in advance in the pattern storage unit 122. However, the CPU 20 may calculate each parameter of an exercise pattern based on physical information on a subject and a predetermined computational expression. Alternatively, only one exercise pattern may be stored in advance.

A breathing exercise method to be performed by the breathing exerciser 100 of the present invention can also be provided in the form of a program. Such a program can also be provided in the form of a program product by storing the program on an optical medium, such as a CD-ROM (Compact Disk-ROM), or in a computer-readable storage medium, such as a memory card. Alternatively, the program can also be provided by download via a network.

A program product to be provided is executed by being installed in a program storage unit such as the memory 12. Note that the program product includes a program itself and a storage medium storing the program.

The embodiments disclosed herein are to be considered in all respects as illustrative and not restrictive. The scope of the present invention is indicated by the appended claims rather than by the foregoing description, and all changes that come within the meaning and range of equivalency of the appended claims are intended to be embraced therein.

**Claims**

1. A breathing exerciser comprising:

   a guide unit (201) for guiding an exercise pattern of breathing to a user;
   a detecting unit for detecting breathing of the user;
   a first calculating unit (203) for calculating, based on a signal from the detecting unit, a breathing index at least one of before and after an exercise period during which the exercise pattern is guided, the breathing index representing a characteristic of a breathing state of the user;
   a second calculating unit (204) for calculating an outcome index based on at least two breathing indices, the outcome index representing an outcome of breathing exercise; and
   an informing unit (206) for informing the outcome index to the user.

2. The breathing exerciser according to claim 1, wherein
   the first calculating unit calculates two breathing indices based on breathings respectively detected before and after the exercise period, and
   the second calculating unit calculates the outcome index for before and after the exercise period, based on the calculated breathing indices.

3. The breathing exerciser according to claim 1 further comprising: a storage unit (126) for storing information on the breathing index which is based on the breathing detected before the previous exercise period, wherein
the first calculating unit calculates the breathing index based on the breathing detected before the current exercise period, and
the second calculating unit calculates the outcome index for before each exercise period, based on the breathing index calculated by the first calculating unit and the information on the breathing index stored in the storage unit.

4. The breathing exerciser according to claim 1 further comprising: a storage unit (126) for storing information on the breathing index which is based on the breathing detected after the previous exercise period, wherein
the first calculating unit calculates the breathing index based on the breathing detected after the current exercise period, and
the second calculating unit calculates the outcome index for after each exercise period, based on the breathing index calculated by the first calculating unit and the information on the breathing index stored in the storage unit.

5. The breathing exerciser according to any one of claims 2 to 4, wherein
the first calculating unit further calculates a plurality of breathing indices based on breathings detected a plurality of times during the exercise period, and
the second calculating unit further calculates the outcome index for during the exercise period, based on the breathing indices for during the exercise period.

6. The breathing exerciser according to claim 5 further comprising: a changing unit (20) for changing the exercise pattern based on a result of comparison between the outcome index for during the exercise period which is calculated by the second calculating unit and a predetermined threshold.

7. The breathing exerciser according to claim 1, wherein the breathing index is one of a breathing cycle or a number of breaths, a balance between an exhalation operation and an inhalation operation, breathing flow rate, uniformity of the breathing flow rate, and silent property.

8. A breathing exerciser comprising:

a guide unit (201) for guiding an exercise pattern of breathing to a user;
a detecting unit (2, 202) for detecting breathing of the user;
a first calculating unit (203) for calculating, based on a signal from the detecting unit, a breathing index at least one of before and after an exercise period during which the exercise pattern is guided, the breathing index representing a characteristic of a breathing state of the user; and
an informing unit (206) for informing at least two breathing indices to the user.

9. The breathing exerciser according to claim 8, wherein the breathing index is one of a breathing cycle or a number of breaths, a balance between an exhalation operation and an inhalation operation, breathing flow rate, uniformity of the breathing flow rate, and silent property.

Fig. 1

Fig. 2

Fig. 3

Fig. 4

126

```
┌─────────────────────────────────────────────┐
│                                             │
│   ┌─────────────────────────────┐           │
│   │ DT1,RCb1, Elb1, Elba1       ├─ R1       │
│   └─────────────────────────────┘           │
│                                             │
│   ┌─────────────────────────────┐           │
│   │ DT2,RCb2, Elb2, Elba2       ├─ R2       │
│   └─────────────────────────────┘           │
│                                             │
│                  │                          │
│                  │                          │
│                                             │
│   ┌─────────────────────────────┐           │
│   │ DTn,RCbn, Elbn, Elban       ├─ Rn       │
│   └─────────────────────────────┘           │
│                                             │
└─────────────────────────────────────────────┘
```

Fig. 5

```
                        ┌─────────┐
                        │  Start  │
                        └─────────┘
                             │
                             ▼         ⌐S2
              ┌──────────────────────────────┐
              │  Accept physical information  │
              └──────────────────────────────┘
                             │
                             ▼         ⌐S4
              ┌──────────────────────────────┐
              │   Determine exercise pattern  │
              └──────────────────────────────┘
                             │
            ┌───────────────▶│         ⌐S6
            │   ┌──────────────────────────────┐
            │   │      Detect breathing for     │
            │   │        before exercise        │
            │   └──────────────────────────────┘
            │                │         ⌐S8
      NO    ◇────────────────────────────────◇
            │   Has predetermined period of
            │        time elapsed?
                            │ YES
                            ▼              ⌐S10
              ┌──────────────────────────────┐
              │  Calculate/store breathing index │
              │       for before exercise      │
              └──────────────────────────────┘
                            │                    ⌐S11
        ┌───────────────────────────────────────────┐
        │  Calculate/inform/store outcome index for │
        │            before each exercise            │
        └───────────────────────────────────────────┘
                            │
          ┌────────────────▶│         ⌐S12
          │   ┌──────────────────────────────┐
          │   │     Guide exercise pattern    │
          │   └──────────────────────────────┘
          │                │         ⌐S14
      NO  ◇────────────────────────────────◇
          │      Has exercise period elapsed?
                           │ YES
          ┌───────────────▶│         ⌐S16
      NO  ◇────────────────────────────────◇
          │     Has predetermined period
          │          of time elapsed?
                           │ YES
          ┌───────────────▶│         ⌐S18
          │   ┌──────────────────────────────┐
          │   │      Detect breathing for     │
          │   │        after exercise         │
          │   └──────────────────────────────┘
          │                │         ⌐S20
      NO  ◇────────────────────────────────◇
          │     Has predetermined period
          │          of time elapsed?
                           │ YES
                           ▼              ⌐S22
        ┌────────────────────────────────────────┐
        │  Calculate breathing index for after exercise │
        └────────────────────────────────────────┘
                           │                    ⌐S24
        ┌────────────────────────────────────────┐
        │  Calculate/inform/store outcome index for │
        │          before and after exercise        │
        └────────────────────────────────────────┘
                           │
                           ▼
                      ┌─────────┐
                      │   End   │
                      └─────────┘
```

Fig. 6

Fig. 7

Fig. 8

Fig. 9

Number of exercises
5th time

Outcome (Number of breaths)

Before exercise
4.2 breaths/min

After exercise
4.0 breaths/min

Fig. 10

Fig. 11

Fig. 12

| **INTERNATIONAL SEARCH REPORT** | International application No. |
|---|---|
| | PCT/JP2006/325904 |

**A. CLASSIFICATION OF SUBJECT MATTER**
*A63B23/18*(2006.01)i, *A61B5/08*(2006.01)i, *A63B24/00*(2006.01)i, *A63B69/00* (2006.01)i, *A61M21/02*(2006.01)n

According to International Patent Classification (IPC) or to both national classification and IPC

**B. FIELDS SEARCHED**

Minimum documentation searched (classification system followed by classification symbols)
A63B23/18, A61B5/08, A63B24/00, A63B69/00, A61M21/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2002-301047 A (Mitsubishi Electric Corp.),<br>15 October, 2002 (15.10.02),<br>Full text<br>(Family: none) | 8-9<br>1-7 |
| Y | JP 7-289540 A (Matsushita Electric Industrial Co., Ltd.),<br>07 November, 1995 (07.11.95),<br>Par. Nos. [0081] to [0082]<br>(Family: none) | 1-7 |
| A | JP 2-271880 A (Kabushiki Kaisha Suzuki Gakki Seisakusho),<br>06 November, 1990 (06.11.90),<br>Claims 1, 3; Fig. 1<br>(Family: none) | 1-9 |

☒ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 March, 2007 (29.03.07) | 10 April, 2007 (10.04.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.

PCT/JP2006/325904

C (Continuation). DOCUMENTS CONSIDERED TO BE RELEVANT

| Category* | Citation of document, with indication, where appropriate, of the relevant passages | Relevant to claim No. |
|---|---|---|
| A | JP 10-43328 A (Masato OSHIKAWA),<br>17 February, 1998 (17.02.98),<br>Claim 1; Fig. 1<br>(Family: none) | 1-9 |

Form PCT/ISA/210 (continuation of second sheet) (April 2005)

<div style="text-align: center;">**INTERNATIONAL SEARCH REPORT**</div>

| International application No. |
| --- |
| PCT/JP2006/325904 |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
The inventions of claims 1-7 relate to a respiration training machine having a first calculating section for calculating a respiration index and a second calculating section for calculating a result index.
The invention of claims 8-9 relate to a respiration training machine having a calculating section for calculating a respiration index.

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**     ☐ The additional search fees were accompanied by the applicant's protest and, where applicable,
the                                payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 62277976 A
- JP 2005535378 W
- JP 2001507364 W
- JP 2005237807 A

**Non-patent literature cited in the description**

- Slow Breathing Improves Arterial Baroreflex Sensitivity and Decreases Blood Pressure in Essential Hypertension. **CHACKO N.JOSEPH et al.** Hypertension. American Heart Association, October 2005, vol. 46, 714-718

- Slow Breathing Improves Arterial Baroreflex Sensitivity and Decreases Blood Pressure in Essential Hypertension. **CHACKO N.JOSEPH ; CESARE PORTA ; GAIA CASUCCI ; NADIA CASIRAGHI ; MARA MAFFEIS ; MARCO ROSSI ; LUCIANO BERNARDI.** Hypertension. American Heart Association, October 2005, vol. 46, 714-718